# EUROPEAN PATENT SPECIFICATION

(11) **EP 3 398 547 B1**
(45) Date of publication and mention of the grant of the patent: **04.10.2023**
(21) Application number: 18162809.0
(22) Date of filing: 20.03.2018
(51) Int. Cl.: A61B 18/02, A61B 18/00

(54) **INTRODUCER AND CRYOPROBE**
EINFÜHRUNGSVORRICHTUNG UND KRYOSONDE
INTRODUCTEUR ET CRYOSONDE

(30) Priority: 28.04.2017 US 201715581574
(43) Date of publication of application: 07.11.2018
(73) Proprietor: Medtronic Holding Company Sàrl, 1131 Tolochenaz (CH)
(72) Inventor: SNYDER, Lloyd M., Collierville, TN Tennessee 38017 (US); KOCH, Brian D., Memphis, TN Tennessee 38103 (US); SAMARANSKA, Aneta, Blaine, MN Minnesota 55449 (US); JUSTIS, Jeff R., Germantown, TN Tennessee 38139 (US)
(74) Representative: Viering, Jentschura & Partner mbB Patent- und Rechtsanwälte

(56) References cited:
- WO-A1-01/13782
- WO-A1-99/65410
- US-A1- 2005 090 779
- US-A1- 2006 264 920
- US-A1- 2008 051 776
- US-A1- 2008 119 838
- US-A1- 2016 287 334
- US-B1- 6 306 129

## Description

The present invention relates to an introducer and a cryoprobe employing the Joule-Thomson effect to generate cryogenic temperatures for use during surgery on a patient. More particularly, the present invention relates to an introducer insertable into the body of a patient and a cryoprobe for use in ablating unwanted tissue of the patient during surgery via freezing the unwanted tissue using cryogenic temperatures generated thereby. The present invention relates to an introducer and a cryoprobe for use during surgery, wherein the introducer is first insertable into the body of a patient, and the cryoprobe is second insertable into the introducer, where cryogenic temperatures generated by the cryoprobe serve in cooling the introducer in order to freeze unwanted tissue around the introducer.

### DESCRIPTION OF THE PRIOR ART

Typically, cryoprobes used for ablation of unwanted tissue during surgery are insertable directly into the patient. That is, the shaft portions of such cryoprobes are inserted into the body of a patient to position end portions thereof adjacent the unwanted tissue. However, such cryoprobes require shaft portions having material thicknesses and strength to withstand the stress of insertion. These properties of such cryoprobes can be disadvantageous in the application of the cryogenic temperatures generated thereby to the unwanted tissues. As such, there is a need for an introducer and a cryoprobe configured for use with one another. Such an introducer can be first inserted into the body of the patient, and such a cryoprobe can be second inserted into the introducer. Using such an introducer and such a cryoprobe affords use of materials for the shafts of the cryoprobes having less thickness and less strength than typical cryoprobes, and affords ablation of the unwanted tissue by cooling of the exterior of such an introducer by such a cryoprobe. From e.g. US 2008/0119838 A1 a combination of a cryoprobe and an introducer is known, including the cryoprobe including a head portion, a probe shaft, and a cryogenic gas supply line and the introducer including a handle portion and a sheath having a closed trocar tip. Further combinations of a cryoprobe and an introducer are known from e.g. US2008/0051776 A1, US 2006/264920 A1, US 6 306 129 B1, WO99/65410 A1 and WO 01/13782 A1.

### SUMMARY OF THE INVENTION

The present invention provides a combination of a cryoprobe and an introducer according to claim 1. Further embodiments of the combination are described in the dependent claims.

The present invention further contemplates a combination of a cryoprobe, an introducer, and a cap portion used for attaching the cryoprobe and the introducer to one another, the combination having the cryoprobe including a head portion, a probe shaft, and a cryogenic gas supply line, the probe shaft having a proximal end and a distal end, the probe shaft being attached to the head portion adjacent the proximal end, the cryogenic gas supply line extending through portions of the head portion and the probe shaft and terminating adjacent the distal end of the probe shaft, the cryogenic gas supply line during operation of the cryoprobe delivering cryogenic gas to the distal end of the probe shaft to facilitate cooling of a portion of the probe shaft; the introducer including a handle portion, a cannula portion, a first internal cavity, and a second internal cavity, the introducer having a proximal end and a distal end, the handle portion provided at the proximal end, and the cannula portion extending from the handle portion to the distal end, the handle portion including the first internal cavity extending therethrough, and the cannula portion including the second internal cavity extending therethrough, the first and second internal cavities communicating with one another and being sized to receive a portion of the probe shaft therethrough; and the cap portion facilitating the interconnection of the cryoprobe and the introducer, the cap portion including a proximal end, a distal end, a body portion provided adjacent the proximal end, a flange portion provided adjacent the distal end, and an internal cavity extending through the cap portion between the proximal end and the distal end thereof, the cap portion including a first attachment mechanism in the internal cavity adjacent the distal end used in attaching the handle portion to the cap portion, and the cap portion including a second attachment mechanism in the internal cavity adjacent the proximal end used in attaching the cryoprobe to the cap portion; where, when the probe shaft is inserted into the first and second internal cavities in the introducer, the distal end of the probe shaft is positionable adjacent the distal end of the introducer, and operation of the cryoprobe facilitates cooling of a portion of the cannula portion at the distal end of the introducer.

The present disclosure further contemplates a method of ablating unwanted tissue during surgery on a patient, the method including providing an introducer partially insertable into the patient, the introducer including a proximal end, a distal end, a handle portion provided adjacent the proximal end, a cannula portion provided extending from the handle portion to the distal end, a first internal cavity extending through the handle portion, and a second internal cavity extending through the cannula portion, the first and second internal cavities communicating with one another; providing a cryoprobe including a head portion, a probe shaft, and a cryogenic gas supply line, the probe shaft having a proximal end and a distal end, the probe shaft being attached to the head portion adjacent the proximal end, the cryogenic gas supply line extending through portions of the head portion and the probe shaft and terminating adjacent the distal end of the probe shaft, inserting a portion of introducer into the patient; positioning the distal end of the introducer adjacent the unwanted tissue; inserting a portion the probe shaft through the first internal cavity and a portion of the second internal cavity of the introducer to position the distal end of the probe shaft in the cannula portion adjacent the distal end of the introducer; delivering cryogenic gas through the cryogenic gas supply line to the distal end of the probe shaft to facilitate cooling of the probe shaft; cooling the cannula portion at the distal end of the introducer; ablating the unwanted tissue by absorbing heat therefrom via the cooling of the cannula portion at the distal end of the introducer; removing the probe shaft from the portion of the second internal cavity and the first internal cavity of the introducer; and removing the introducer from the patient.

These and other objects of the present invention will be apparent from review of the following specification and the accompanying drawings.

### BRIEF DESCRIPTION OF THE DRAWINGS

FIG. 1 is a perspective view a cryoprobe according to an embodiment of the present invention;
FIG. 2 is a side elevational view of the cryoprobe of FIG. 1;
FIG. 3 is an enlarged view of an end portion of the cryoprobe of FIG. 1;
FIG. 4 is a cross-sectional view of the end portion of the cryoprobe taken along Line 4-4 of FIG. 3;
FIG. 5 is an enlarged view of a head portion of the cryoprobe of FIG. 1;
FIG. 6 is a cross-sectional view of the head portion of the cryoprobe taken along Line 6-6 of FIG. 5;
FIG. 7 is an enlarged cross-sectional view of a portion of the head portion and a portion of a probe portion of the cryoprobe of FIG. 6;
FIG. 8 is an enlarged cross-sectional view of yet another portion of the head portion of FIG. 6;
FIG. 9 is an enlarged cross-sectional view of another portion of the head portion and another portion of the probe portion of the cryoprobe of FIG. 6;
FIG. 10 is an enlarged cross-sectional view of yet another portion of the probe portion of the cryoprobe of FIG. 6;
FIG. 11 is an enlarged cross-sectional view of the interior of an exterior tube of the cryoprobe of FIG. 1;
FIG. 12 is a first perspective view of an introducer according to an embodiment of the present invention;
FIG. 13 is a second perspective view of the introducer of FIG. 12;
FIG. 14 is a top plan view of the introducer of FIG. 12;
FIG. 15 is a side elevational view of the introducer of FIG. 12;
FIG. 16 is a cross-sectional view of the introducer taken along Line 16-16 of FIG. 15;
FIG. 17 is an exploded view of the introducer of FIG. 12 and the cryoprobe of FIG. 1;
FIG. 18 is a partial assembled view of the introducer of FIG. 12 and the cryoprobe of FIG. 1 showing the introducer being inserted into the body of a patient;
FIG. 19 is a side elevational view of the introducer of FIG. 12 and the cryoprobe of FIG. 1 assembled together; and
FIG. 20 is a cross-sectional view of the assembled introducer and cryoprobe of FIG. 20.

### DETAILED DESCRIPTION OF THE PREFERRED EMBODIMENT

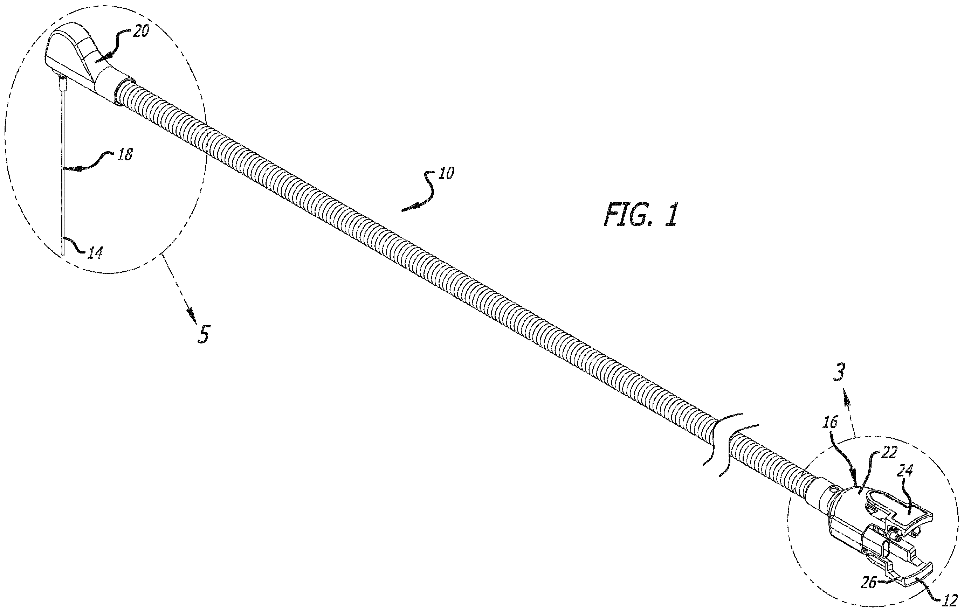

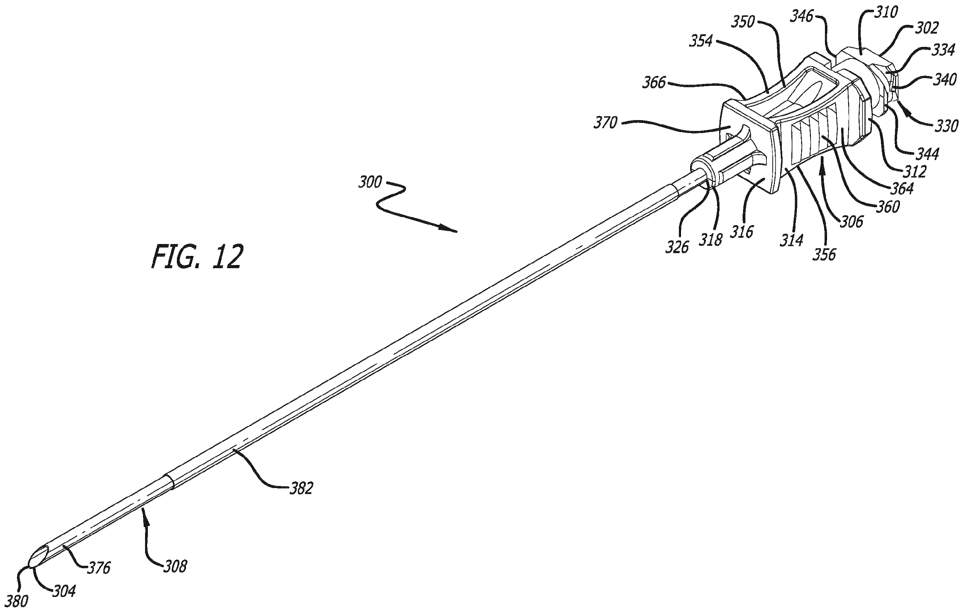

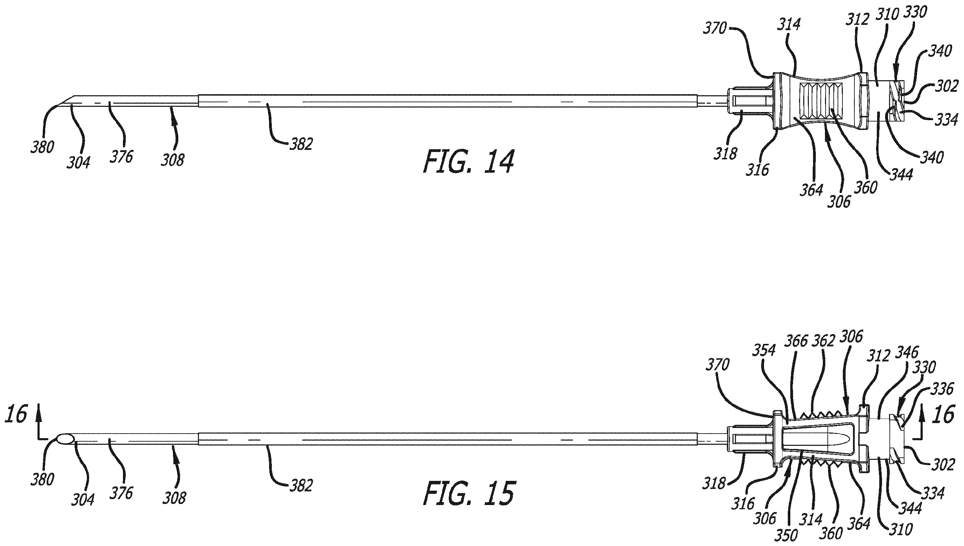

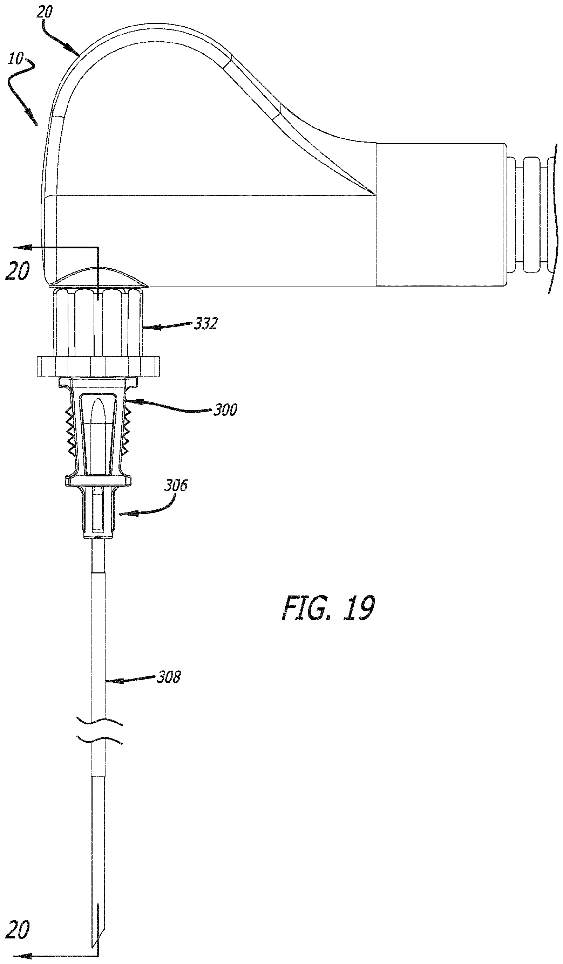

In accordance with one preferred embodiment of the present invention, and, as depicted in FIGS. 1-11 and 17-20, a cryoprobe generally indicated by the numeral 10 is provided for use during surgery to facilitate ablation of unwanted tissue inside the body of a patient. Furthermore, in accordance with the preferred embodiment of the present invention, and, as depicted in FIGS. 12-20, an introducer generally indicated by numeral 300 is provided for use during surgery to at the very least facilitate placement of the cryoprobe 10 with respect to the unwanted tissue inside the body of the patient.

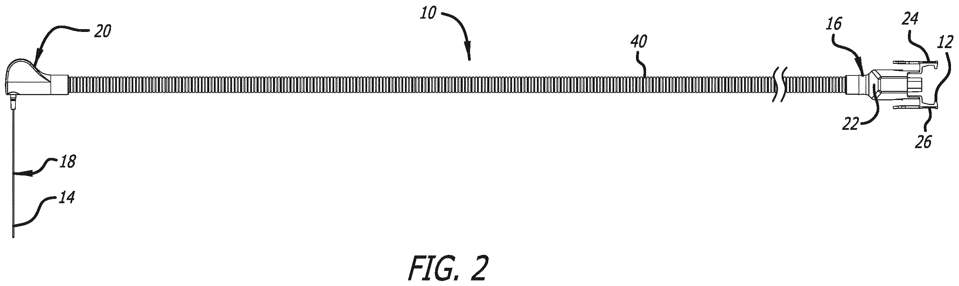

The cryoprobe 10, as depicted in FIGS. 1 and 2, includes a proximal end 12 and a distal end 14 opposite from one another. As discussed below, the cryoprobe 10 includes an end portion 16 provided at the proximal end 12, a probe portion 18 provided at the distal end 14, and a head portion 20 positioned between the proximal end 12 and the distal end 14. The various components of the cryoprobe 10 can be made of metallic and polymeric materials. However, it is noted that polymeric materials can be used where insulative properties are desirous, and the metallic materials can be used where heat transfer properties are desirous. Furthermore, the tubes and the supply/return lines discussed below can be cylindrical to facilitate ease of construction, but the tubes and the supply/return lines can have other shapes and configurations.

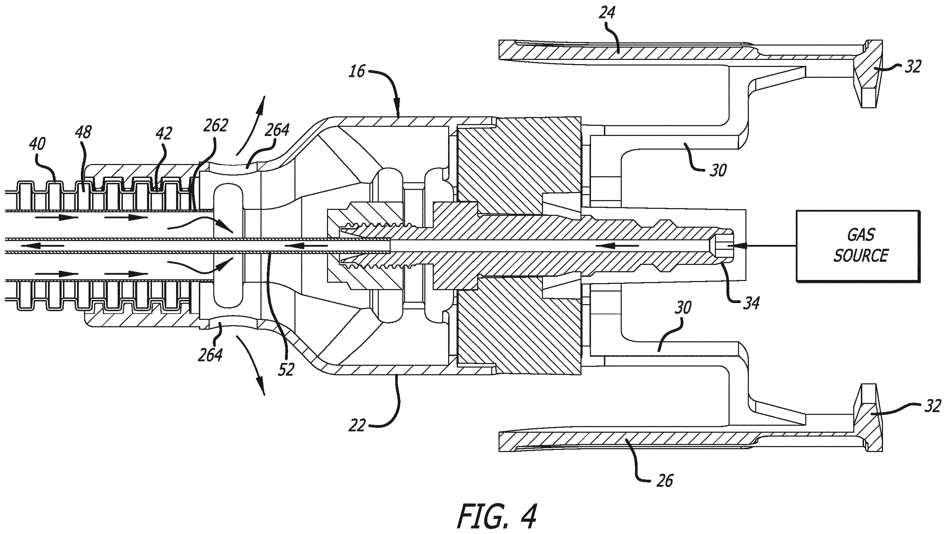

To facilitate ablation of unwanted tissue, a portion of the cryoprobe 10 is inserted into the body of the patient. The cryoprobe 10 is capable of generating external cryogenic temperatures (e.g., ranging from -80 to -120 °C) on an exterior portion of the probe portion 18 adjacent the distal end 14, and thus, the portion of the probe portion 18 serves as a heat exchanger to facilitate ablation of the unwanted tissue via freezing thereof using the cryogenic temperatures. In doing so, the cryoprobe 10 is capable of generating internal cryogenic temperatures (e.g., ranging from -100 °C to - 150 °C). For example, a surgeon can use the cryoprobe 10 to surgically ablate cancerous tumors via the freezing thereof. As discussed below, the cryoprobe 10 employs the Joule-Thomson effect to generate the cryogenic temperatures in the probe portion 18. To that end, the cryoprobe 10 uses a supply of cryogenic gas from a cryogenic gas supply (not shown) that can be turned on and off as needed. The flow of cryogenic gas through the cryoprobe 10 is indicated by various arrows in FIGS. 4 and 7-11. Supply gas travels from the cryogenic gas supply through the cryoprobe from the proximal end 12 to the distal end 14, and return gas travels from the distal end 14 to the proximal end 12.

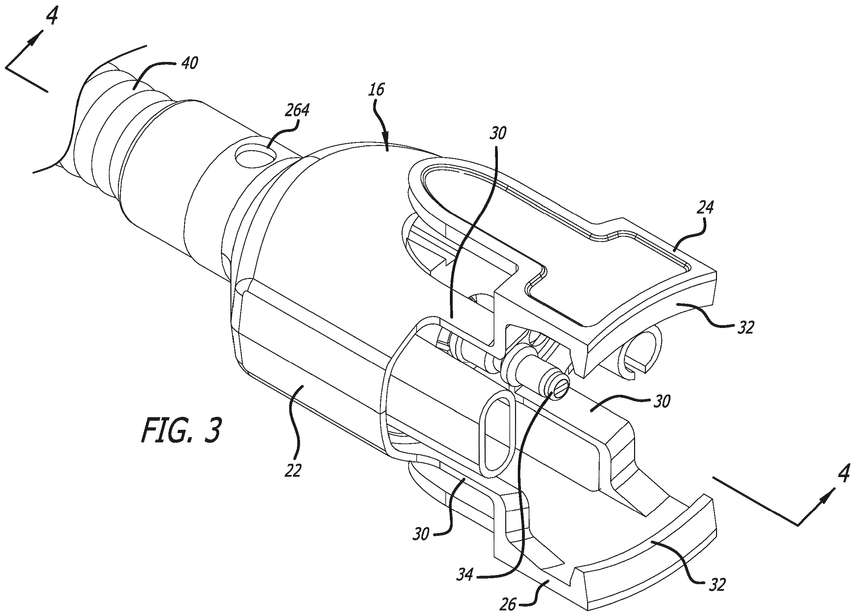

The cryoprobe 10 includes an end portion 16 provided at the proximal end 12 for facilitating interconnection with the cryogenic gas supply. As depicted in FIGS. 3 and 4, the end portion 16 includes a body portion 22, and a first arm portion 24 and a second arm portion 26 attached to the body portion 22. The first and second arm portions 24 and 26 are each attached to the body portion 22 via connecting portions 30, and each of the first and second arm portions 24 and 26 include latching portions 32. The connecting portions 30 allow the first and second arm portions 24 and 26 to pivot with respect to the body portion 22, and such pivotal movement allows the latching portions 32 to move inwardly and outwardly with respect to one another. The latching portions 32 can be used to engage complementary structures provided on the cryogenic gas supply, and such engagement can serve to connect the cryoprobe 10 to the cryogenic gas supply.

In addition to facilitating attachment to the cryogenic gas supply, the end portion 16 also includes an inlet connector 34 for engaging a complementary structure (not shown) on the cryogenic gas supply. As depicted in FIG. 4, the inlet connector 34 extends through the end portion 16. The inlet connector 34 facilitates connection of the cryoprobe 10 to a supply of gas provided by the cryogenic gas supply. The supply gas provided by the cryogenic gas supply can include Argon, Krypton, Xenon, CO2, N2O, and N2, for example. The supply gas provided by the cryogenic gas supply is provided at a high pressure (e.g., ranging from 3000 to 3400 psi) (20.7 - 23.4 MPa) to facilitate use of the Joule-Thomson effect by the cryoprobe 10.

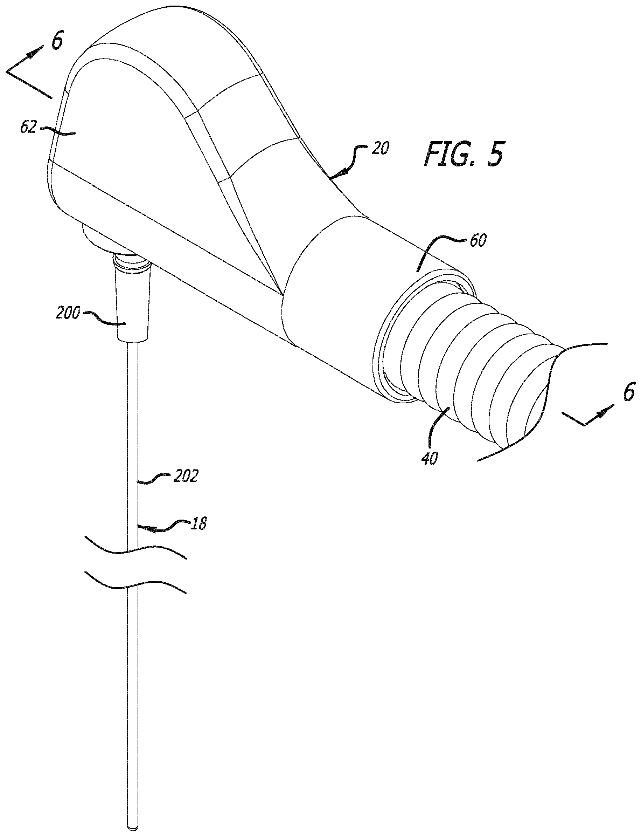

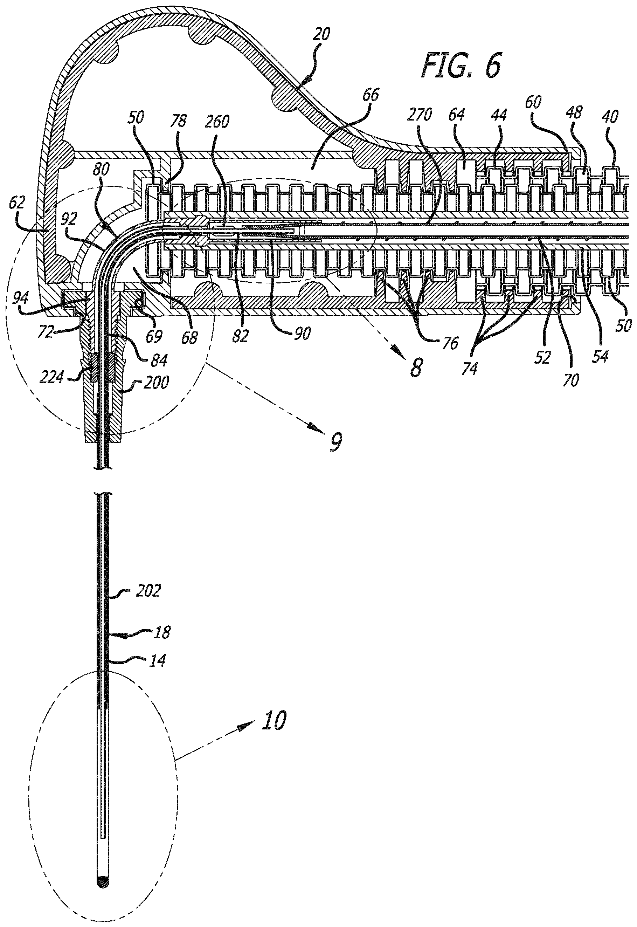

As depicted in FIGS. 3-6 and 11, the cryoprobe 10 further includes an exterior tube 40 having a first end 42 and a second end 44. The first end 42 of the exterior tube 40 is attached (FIGS. 3 and 4) to the end portion 16, and the second end 44 of the exterior tube 40 is attached to the head portion 20 (FIGS. 5 and 6). The exterior tube 40 includes an interior 48, and the interior 48 contains internal structures that facilitate transfer of the supply gas from the end portion 16 to the head portion 20, and contains internal structures that facilitate transfer of return gas from the head portion 20 to the end portion 16. The exterior tube 40 affords protection of the internal structures, and the exterior tube 40 is flexible. The exterior tube 40 can be corrugated or convoluted to afford such flexibility. The flexibility of the exterior tube 40 affords manipulation thereof during surgery, and thus, the exterior tube 40 affords positioning and repositioning of the head portion 20.

The interior 48 of the exterior tube 40, as depicted in FIG. 6, includes an interior tube 50, a first gas supply line 52, and a first gas return line 54 extending therethrough. Like the exterior tube 40, the interior tube 50, the first gas supply line 52, and the first gas return line 54 can be flexible. The interior tube 50 can be corrugated or convoluted to afford such flexibility, and the first gas supply line 52 and the first gas return line 54 can be made of flexible materials. The first gas supply line 52 is interconnected with the inlet connector 34 (FIG. 4), and is used in facilitating passage of the supply gas to adjacent the distal end 14. Specifically, the first gas supply line 52 is configured to transfer the supply gas from the cryogenic gas supply to the head portion 20. Furthermore, the first gas return line 54 is used in facilitating passage of the return gas to adjacent the proximal end 12. The first gas supply line 52 is received inside the first gas return line 54, the first gas return line 54 is received inside the interior tube 50, and the interior tube 50 is received inside the exterior tube 40. Like the exterior tube 40, the interior tube 50 is flexible, and can be corrugated or convoluted and made of semi- rigid polymeric and/or polymeric materials to afford protection of the internal structures, where the corrugation or convolution thereof can afford such flexibility. The flexibility of the exterior tube 40, the interior tube 50, the first gas return line 54, and the first gas supply line 52 affords manipulation thereof during surgery to afford positioning and repositioning of the head portion 20.

The head portion 20, as depicted in FIGS. 5 and 6, includes a first end portion 60 and a second end portion 62. As depicted in FIG. 6, the head portion 20 is hollow, and includes at least a first internal cavity 64, a second internal cavity 66, a third internal cavity 68, and a fourth internal cavity 69. The first end portion 60 includes a first aperture 70 communicating with the first internal cavity 64 and sized to receive the exterior tube 40 (and portions of the interior tube 50, the first gas supply line 52, and the first gas return line 54) therethrough. The second end portion 62 includes a second aperture 72. Furthermore, the first internal cavity 64 includes a first set of internal ribs 74 that are sized to complement the corrugation of the exterior tube 40. To attach the exterior tube 40 to the head portion 20, a portion of the exterior tube 40 is inserted through the first aperture 70 into the first internal cavity 64, so that at least a portion of the first set of internal ribs 74 are press fit into the corrugation of the exterior tube 40. Such a press fit serves in holding the exterior tube 40 in position relative to the head portion 20.

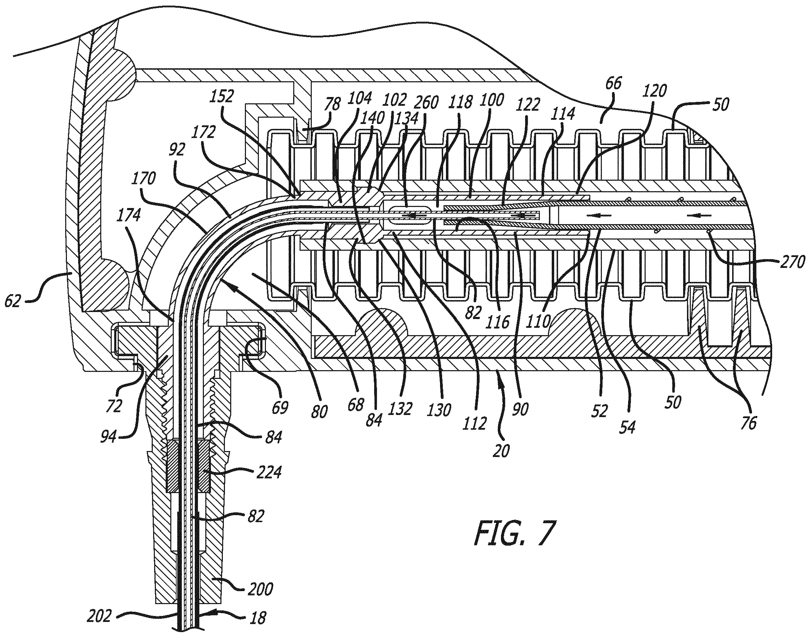

The interior tube 50 is also attached to the head portion 20 (FIGS. 6 and 7). In addition to the first set of internal ribs 74, the first internal cavity 64 also includes a second set of internal ribs 76, and a shoulder portion 78 is provided between the second internal cavity 66 and the third internal cavity 68. To attach the interior tube 50 to the head portion 20, a portion of the interior tube 50 is inserted through the first aperture 70 into the first internal cavity 64, the second internal cavity 66, and past the shoulder portion 78, so that at least a portion of the second set of internal ribs 76 and the shoulder portion 78 are press fit into the corrugation of the interior tube 50. Such a press fit serves in holding the interior tube 50 in position relative to the head portion 20.

The head portion 20 also includes a transition portion 80. The transition portion 80, as depicted in FIGS. 6-9, is provided to effectuate coupling of the first gas return line 54 to a second gas return line 84. The transition portion 80 includes a first portion 90, a second portion 92, and a third portion 94. The first portion 90 of the transition portion 80 is received on the inside of the first gas return line 54, and is formed from a first sleeve portion 100, a flange portion 102, and a second sleeve portion 104 attached to one another.

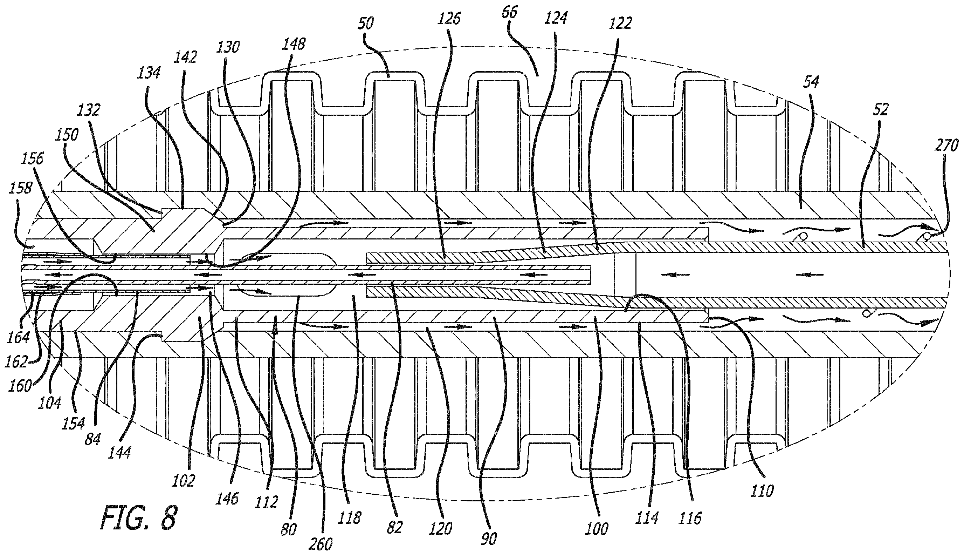

As depicted in FIGS. 7 and 8, the first sleeve portion 100 can be cylindrical, and includes a first end 110 and a second end 112. The first sleeve portion 100 also includes an exterior surface 114, an interior surface 116, and an internal cavity 118 extending between the first end 110 and the second end 112. The external dimensions of the first sleeve portion 100 (as defined by the exterior surface 114) are slightly smaller than the internal dimensions of the first gas return line 54. As such, a gap 120 is formed between the exterior surface 114 of the first sleeve portion 100 and the internal dimensions of the first gas return line 54. As discussed below, the transition portion 80 and the gap 120 (between the first gas return line 54 and the transition portion 80) are used in facilitating passage of the return gas to adjacent the proximal end 12.

The internal dimensions of the internal cavity 118 of the first sleeve portion 100 (as defined by the interior surface 116) are sized to receive the first gas supply line 52 therein. For example, the internal dimensions of the internal cavity 118 can be sized to complement the external dimensions of the first gas supply line 52. The fit between the first gas supply line 52 and the internal cavity 118 can also be fluid tight. The fluid- tight fitment between the first gas supply line 52 and the internal cavity 118 can be effectuated by crimping of the first gas supply line 52 and first sleeve portion 100 together. As such, the fit between the first gas supply line 52 and the internal cavity 118 can serve in attaching the first gas supply line 52 to the transition portion 80.

The first gas supply line 52, as depicted in FIGS. 6-8, is attached the second gas supply line 82 inside the internal cavity 118 of the first sleeve portion 100. The first gas supply line 52 includes an end portion 122 that necks down from the remainder thereof. To the end, the end portion 122 can include a frusto-conical portion 124 and a cylindrical portion 126. The frusto-conical portion 124 serves in transitioning the remainder of the first gas supply line 52 to the cylindrical portion 126. The internal dimensions of the end portion 122 are sized to receive the second gas supply line 82 therein. For example, the internal dimensions of the cylindrical portion 126 can be sized to complement the external dimensions of the second gas supply line 82. As such, the fit between the second gas supply line 82 and the cylindrical portion 126 can serve in attaching the second gas supply line 82 to the first gas supply line 52. The fit between the second gas supply line 82 and the first gas supply line 52 can be fluid tight. The fluid-tight fitment between the second gas supply line 82 and the first gas supply line 52 can be effectuated by welding or brazing. As discussed below, the second gas supply line 82 extends from the transition portion 80 and terminates in the probe portion 18. The second gas supply line 82, like the first gas supply line 52, is used in facilitating passage of the supply gas to adjacent the distal end 14.

As depicted in FIGS. 7 and 8, the flange portion 102 includes a first end 130 and a second end 132. The flange portion 102 also includes an exterior surface 134 having exterior dimensions configured to be press-fit into the first gas return line 54 and the fit between the flange portion 102 and the first gas return line 54 can be fluid tight. Furthermore, the exterior surface 134 can include a leading surface 142 (adjacent the first end 130) and a trailing surface 144 (adjacent the second end 132) for facilitating the press-fit of the flange portion 102 into the first gas return line 54. To illustrate, the leading surface 142 is angled to facilitate insertion of the flange portion 102 into the first gas return line 54, and the trailing surface 144 serves in preventing withdrawal of the flange portion 102 from the first gas return line 54.

The flange portion 102 also includes an internal cavity 146 having an interior surface 148 extending therethrough. The internal dimensions of the internal cavity 146 (as defined by the interior surface 148) are sized to receive the second gas return line 84 therein. As discussed below, the second gas return line 84 extends from the transition portion 80 and terminates in the probe portion 18, and the second gas supply line 82 extends through second gas return line 84 along its length. The second gas return line 84, like the first gas return line 54, is used in facilitating passage of the return gas to adjacent the proximal end 12.

As depicted in FIGS. 7-9, the second sleeve portion 104 can be cylindrical, and includes a first end 150 and a second end 152. The second sleeve portion 104 also includes an exterior surface 154, a first internal cavity portion 156, and a second internal cavity portion 158. The exterior dimensions of the second sleeve portion 104 (as defined by the exterior surface 154) are sized to correspond to the interior dimensions of the first gas return line 54. As such, the fit between the second sleeve portion 104 and the first gas return line 54 can be fluid tight.

Together, the first internal cavity portion 156 and the second internal cavity portion 158 extend between the first end 150 and the second end 152 of the second sleeve portion 104. The first internal cavity portion 156 includes an interior surface 160. The first internal cavity portion 156 communicates with the internal cavity 146, and like the internal cavity 146, the internal dimensions of the first internal cavity portion 156 (as defined by the interior surface 160) are sized to receive the second gas return line 84 therein.

Furthermore, the second internal cavity portion 158 is sized at least to afford passage of the second gas return line 84 therethrough. The second internal cavity portion 158 is also sized to afford passage of an insulating sheath 162 that surrounds a portion of the second gas return line 84. The insulating sheath 162, as discussed below, is attached to and surrounds the portion of the second gas line 84 to create an insulative cavity 164. The insulative cavity 164, for example, can be filled with an insulative material and/or gas to insulate against the cooling effect of the cold gas traveling through the second gas return line 84. For example, the insulating sheath 162 can be formed for Insulon^{®} from Concept Group, Inc., and the insulative cavity 164 can be a pulled vacuum. As such, the insulative cavity 164 serves in isolating the second gas return line 84 (and the second gas supply line 82 running therethrough) from various portions of the cryoprobe 10.

As discussed above, the second gas supply line 82 and the second gas return line 84 extend from the translation portion 80 and terminate in the probe portion 18. As such, the second portion 92 and the third portion 94 of the transition portion 80 are configured to afford passage of the second gas supply line 82 and the second gas return line 84, as well as the insulating sheath 162, therethrough.

As depicted in FIGS. 6, 7, and 9, the second portion 92 of the transition portion 80 extends through the third internal cavity 68 of the head portion 20. The second portion 92 is formed from a tubular elbow portion 170, and includes a first end 172 and a second end 174. The second portion 92 is attached at the first end 172 to the first portion 90, and attached at the second end 174 to the third portion 172. Furthermore, the tubular elbow portion 170 includes an internal cavity 176 extending between the first end 172 and the second end 174. The internal cavity 176 communicates with the internal cavities extending through the first portion 90 (including the internal cavity 118, the internal cavity 146, the first internal cavity portion 156, and the second internal cavity portion 158). The internal cavity 176 is sized at least to afford passage of the insulating sheath 162 therethrough.

As depicted in FIGS. 6, 7, and 9, the third portion 94 of the transition portion 80 extends from the inside to the outside of the head portion 20. The third portion 94 is formed from a flange portion 180 and a tubular portion 182 attached to one another, and includes a first end 184 and a second end 186. The third portion 94 is attached at the first end 184 to the second portion 92, and, as discussed below, the tubular portion 182 is threaded to receive a portion of the probe portion 18 thereon. Furthermore, the flange portion 180 and the tubular portion 182 includes an interior cavity 188 extending between the first end 184 and the second end 186. The internal cavity 188 communicates with the internal cavities extending through the first portion 90 (including the internal cavity 118, the internal cavity 146, the first internal cavity portion 156, and the second internal cavity portion 158) and the second portion 92 (including the internal cavity 176). The internal cavity 188 is sized at least to afford passage of the insulating sheath 162 therethrough.

The probe portion 18, as depicted in FIGS. 6, 7, and 9, includes a coupler portion 200 and a probe shaft 202. The coupler portion 200 facilitates attachment of the probe portion 18 to the head portion 20, and the probe shaft 202 is configured for insertion into the body of the patient. The coupler portion 200 is formed from a flange portion 204 and a body portion 206, and includes a first end 208 and a second end 210. The flange portion 204 is configured to be received on the interior of head portion 20 in the fourth cavity 69.

The coupler portion 200 includes a first internal cavity portion 212, a second internal cavity portion 213, a third internal cavity portion 214, a fourth internal cavity portion 215, and a fifth internal cavity portion 216. The first internal cavity portion 212 extends through the flange portion 204 and a portion of the body portion 206, and the second internal cavity portion 213, the third internal cavity portion 214, the fourth internal cavity portion 215, and the fifth internal cavity portion 216 extend through the body portion 206. A first opening 220 communicating with the first internal cavity portion 212 is provided in the flange portion 204 at the first end 208 of the coupler portion 200, and a second opening 222 communicating with the fifth internal cavity portion 216 is provided in the body portion 206 at the second end 210.

The second internal cavity portion 213 is threaded to complement the threads provided on the tubular portion 182 of the third portion 94. As such, the tubular portion 182 can be received in second cavity portion 213, and the threads thereof can be engaged to attach the probe portion 18 to the transition portion 80. When the threads of the tubular portion 182 and the second cavity portion 213 are engaged, the flange portion 180 of the third portion 94 is received in the first internal cavity 212 of the coupler portion 200.

The second internal cavity portion 213 and the third internal cavity portion 214 are sized to receive an internal bushing 224 to support the passage of the insulating sheath 162 (and hence, the second gas return line 84 and the second gas supply line 82) during passage thereof through the coupler portion 200. The internal bushing 224 includes a passage 226 therethrough. The passage 226 can be sized to complement the external dimensions of the insulating sheath 162, and the insulating sheath 162 and internal bushing 224 can be welded or brazed to one another therein to facilitate a connection therebetween. As such, the connection between the insulating sheath 162 can serve in holding the insulating sheath 162 in position as it extends through the coupler portion 200. Furthermore, the internal bushing 224 can be "sandwiched" between the second end 186 of the third portion 94 of the transition portion 80 and an internal shoulder 228 formed in the coupler portion 200.

The fourth internal cavity portion 215 and the fifth internal cavity portion 216 are sized to receive a portion of the probe shaft 202 therein, and the probe shaft 202 extends outwardly from the coupler through the second opening 222. For example, the internal dimensions of the fifth internal cavity portion 216 can be sized to complement the external dimensions of the probe shaft 202. The fit between the probe shaft 202 and the fifth internal cavity portion 216 can be fluid tight. The fluid-tight fitment between the probe shaft 202 and the fifth internal cavity portion 216 can be effectuated by welding or brazing. As such, the fit between the probe shaft 202 and the fifth internal cavity portion 216 can serve in attaching the probe shaft 202 to the coupler portion 200.

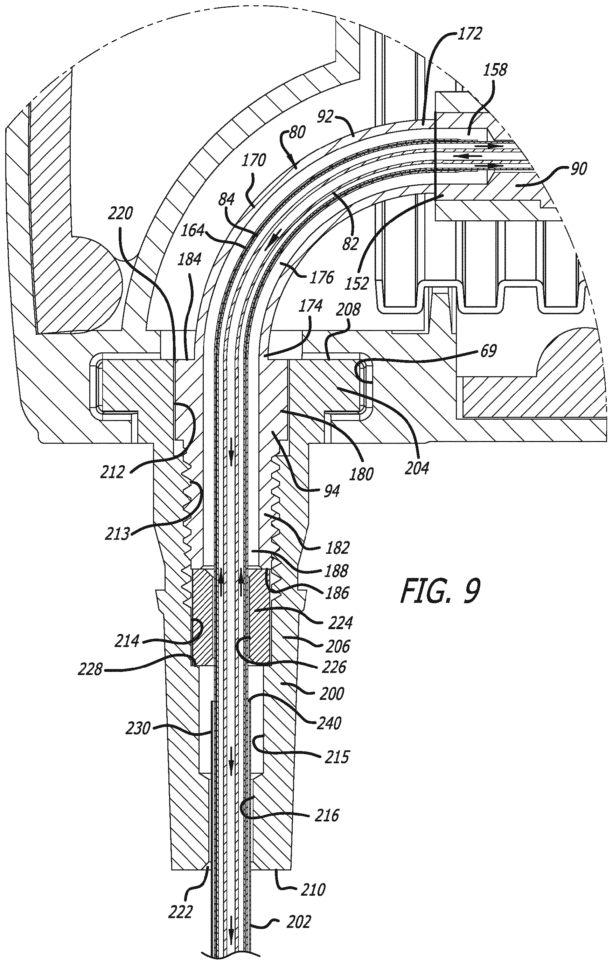

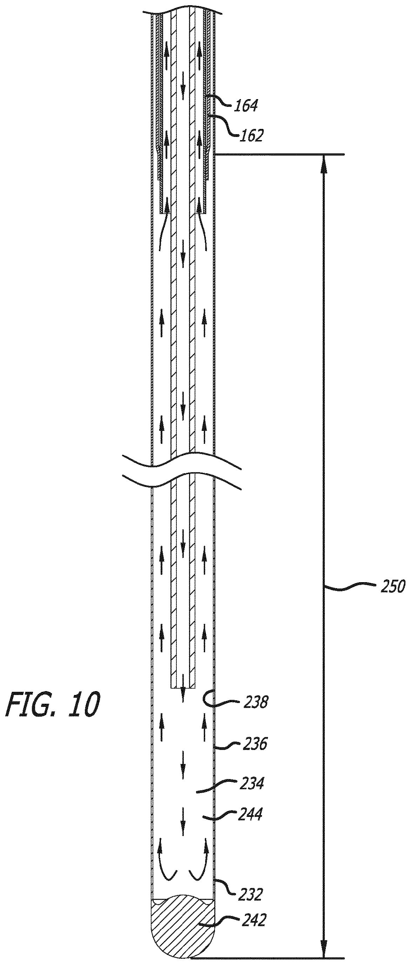

As depicted in FIGS. 9 and 10, the probe shaft 202 includes a first end 230 and a second end 232, and the second end 232 corresponds to the distal end 14 of the cryoprobe 10. Furthermore, the probe shaft 202 is hollow and includes an internal cavity 234. The probe shaft 202 includes an exterior surface 236 and an interior surface 238 includes an opening 240 at the first end 230, and a tip 242 at the second end 232. The internal cavity 234 and the opening 240 are sized to receive the insulating sheath 162 (and hence, the second gas return line 84 and the second gas supply line 82) therethrough. Furthermore, the insulating sheath 162, the second gas return line 84, and the second gas supply line 82 terminate inside the internal cavity 234. As depicted in FIG. 10, the second gas supply line 82 extends beyond the second gas return line 84, and the second gas return line 84 extends beyond the insulating sheath 162. An expansion area 244 in the internal cavity 234 adjacent the terminal end of the second gas supply line 82 is provided to afford operation of the Joule-Thomson effect. As depicted in FIG. 10, the supply gas exiting the second gas supply line 82 enters the expansion area 244.

The supply gas is supplied to the expansion area 244 from the cryogenic gas supply through the cryoprobe 10 via travel through the first gas supply line 52 and the second gas supply line 82. As discussed above, the first gas supply line 52 and the second gas supply line 82 traverse various components of the cryoprobe 10. To illustrate, the first gas supply line 52 extends from the end portion 16 through the first gas return line 54 and a portion of the transition portion 80. The second gas supply line 82 is connected to the first gas supply line 52 in the transition portion 80. From the connection with the first gas supply line 52, the second gas supply line 82 extends through the transition portion 80 and enters the second gas return line 84 inside the transition portion 80. The second gas return line 84 with the second gas supply line 84 received therein extends through portions of the transition portion 80, and then through portions of the probe portion 18. As discussed above, the second gas supply line 82 and the second gas return line 84 terminate in the probe 202 adjacent the distal end 14.

As discussed above, the supply gas is provided at a high pressure (e.g., ranging from 3000 to 3400 psi). (20.7 - 23.4 MPa) The expansion of the supply gas entering the expansion area 244 immediately decreases in temperature due to the Joule-Thomson effect. As such, the supply gas is further cooled by the Joule-Thomson effect. The cryogenic temperatures of the cooling gas in the expansion area 244 cools an end portion 250 of the probe shaft 202. The end portion 250 of the probe shaft 202 can be made of a material facilitating transfer of the cryogenic temperatures thereto. As discussed above, the cryoprobe 10 can be used to ablate unwanted tissue in the body of the patient. To that end, the end portion 250 can be positioned adjacent the unwanted tissue (such as a cancerous tumor) that is to be ablated. The cryogenic temperatures generated at the end portion 250 facilitates heat transfer from the adjacent tissue, and in doing so, freezes the unwanted tissue. An ice ball of frozen unwanted tissue forms around the end portion 250. Freezing in this manner serves to ablate the unwanted tissue.

As depicted in FIGS. 8-10, after the supply gas is supplied to the expansion area 244, the return gas is returned to adjacent the end portion 16 through the cryoprobe 10 via travel through the second gas return line 84, the transition portion 80, and the first gas return line 54. As discussed above, the second gas supply line 84, the transition portion 80, and the first gas return line 54 traverse various components of the cryoprobe 10. To illustrate, the second gas return line 84 extends through a portion of the probe shaft 202 and the coupler portion 200. From the coupler portion 200, the second gas return line 84 extends through portions of the transition portion 80. The return gas travels through the path of the second gas return line 84 and around the second gas supply line 82 to arrive in the internal cavity 118 of the first sleeve portion 100 of the transition portion 80.

The first sleeve portion 100 includes an aperture 260 formed therein between the interior surface 116 and the exterior surface 114 thereof. As depicted in FIGS. 6-8, the aperture 260 affords passage of the return gas from the internal cavity 118 into the gap 120. Additional apertures (not shown) can be provided in the first sleeve portion 100 to afford passage of the return gas from the internal cavity 118 into the gap 120. For example, additional apertures can be spaced 90°, 180°, and 270° apart from the aperture 260. After exiting the aperture 260, the return gas enters the gap 120 between the first gas return line 54 and the first sleeve portion 100. From the gap 120, the return gas travels through the path of the first gas return line 54 and around the first gas supply line 52 to arrive at the end of the first gas return line 54.

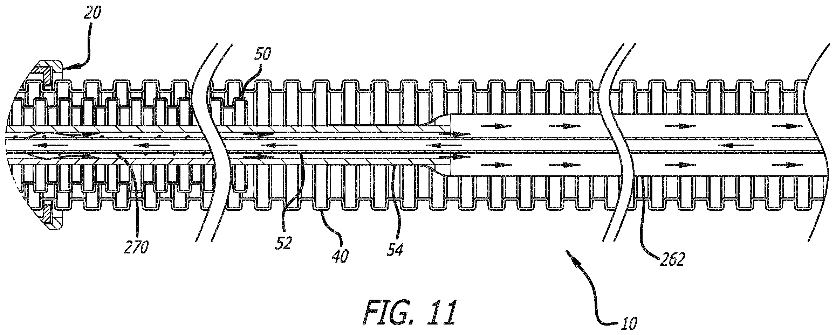

As depicted in FIG. 11, the end of the first gas return line 54 is attached to an expansion tube 262. The expansion tube 262 extends between the end of the first gas return line 54 and the end portion 16. The expansion tube 262 serves as a muffler to decrease the noise of the return gas traveling through the cryoprobe 10. The expansion tube 262 communicates with an exit aperture 264 formed in the end portion 16, and the return gas can be expelled from the cryoprobe 10 through the exit aperture 264. Like the exterior tube 40, the interior tube 50, the first gas return line 54, and the first gas supply line 52, the expansion tube 262 can be flexible. The flexibility of the exterior tube 40, the interior tube 50, the first gas return line 54, the first gas supply line 52, and the expansion tube 262 affords manipulation thereof during surgery to afford positioning and repositioning of the head portion 20.

Flow of the return gas during travel thereof through the cryoprobe 10 can be used to precool the supply gas. As discussed above, the return gas travels around the second gas supply line 82 as it travels through the second gas return line 84, and travels around the first gas supply line 52 as it travels through the first gas return line 54. During such travel around the second gas supply line 82 and the first gas supply line 52, the return gas can be used to precool the supply gas traveling through the second gas supply line 82 and the first gas supply line 52, respectively. To increase the heat exchanging effects of the colder return gas on the supply gas, the first gas supply line 52, the second gas supply line 82, and the second gas return line 84 can be made of metallic materials to facilitate heat transfer between the supply gas and the return gas. Furthermore, to additionally increase the heat exchanging effects of the colder return gas on the supply gas, the spaces between the second gas supply line 82 and the second gas return line 84 and between the first gas supply line 52 and the first gas return line 54 can include turbulence inducing structures to increase turbulence in the flow of the return gas. Increased turbulence in the flow of the return gas insures contact of the return gas with the second gas supply line 82 and the first gas supply line 52, and such contact of the colder return gas serves to remove heat from the supply gas flowing through the second gas supply line 82 and the first gas supply line 52. For example, the space between the first gas supply line 52 and the first gas return line 54 can be provided with a turbulence inducer 270. A similar turbulence inducer can also be provided in the space between the second gas supply line 82 and the second gas return line 84. The turbulence inducer 270 has a helical structure wrapped around the first gas supply line 52 that induces eddy currents in the return gas to increase contact of the return gas with the first gas supply line 52. The turbulence inducer 270 can also be formed as baffles and/or protrusions such as bumps, fins, and/or ribs formed on the exterior surface of the first gas supply line 52.

In addition to the insulating sheath 162 (and the insulative cavity 164 formed in part thereby), the gaps between exterior tube 40, the interior tube 50, the first gas return line 54, and the expansion tube 262 serve in insulating these portions of the cryoprobe 10 from the warming by the outside environment and against the cooling effect of the cooling gas traveling through the cryoprobe 10. Additionally, the gaps between the insulating sheath 162 and the interior surfaces of the second internal cavity portion 158 (of the first portion 90), the internal cavity 176 (of the second portion 92), and the internal cavity 188 (of the third portion 94) serve in insulating these portions of the cryoprobe 10 from the warming by the outside environment and against the cooling effect of the cooling gas traveling through the cryoprobe 10. Similarly, the first internal cavity 64, the second internal cavity 66, the third internal cavity 68, and other internal cavities in the head portion 20 serve to insulate the cryoprobe 10 from the heat from a user's hand and from the cooling effect of the cooling gas traveling through the cryoprobe 10.

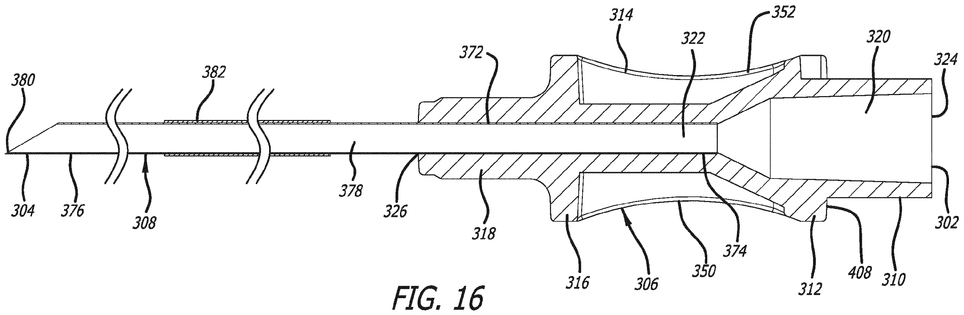

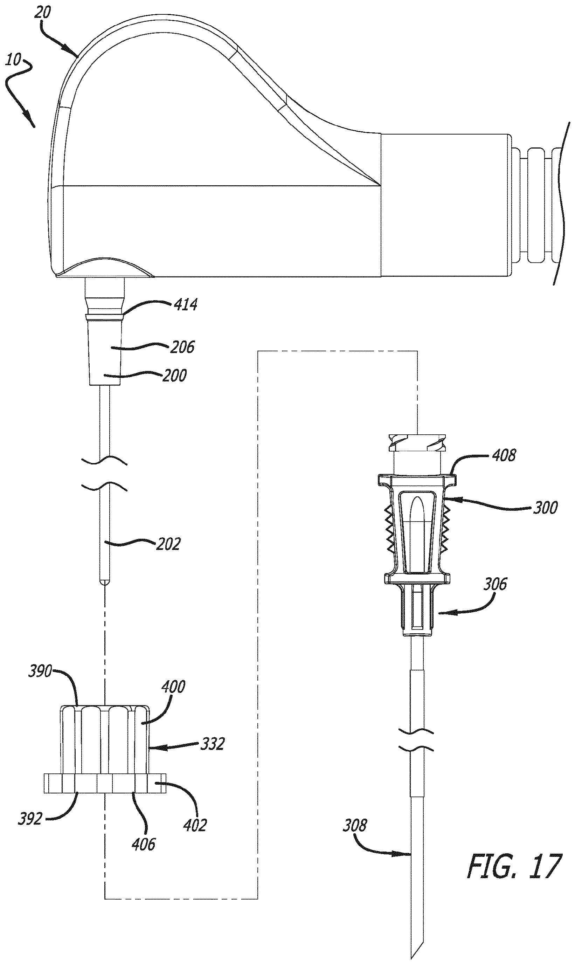

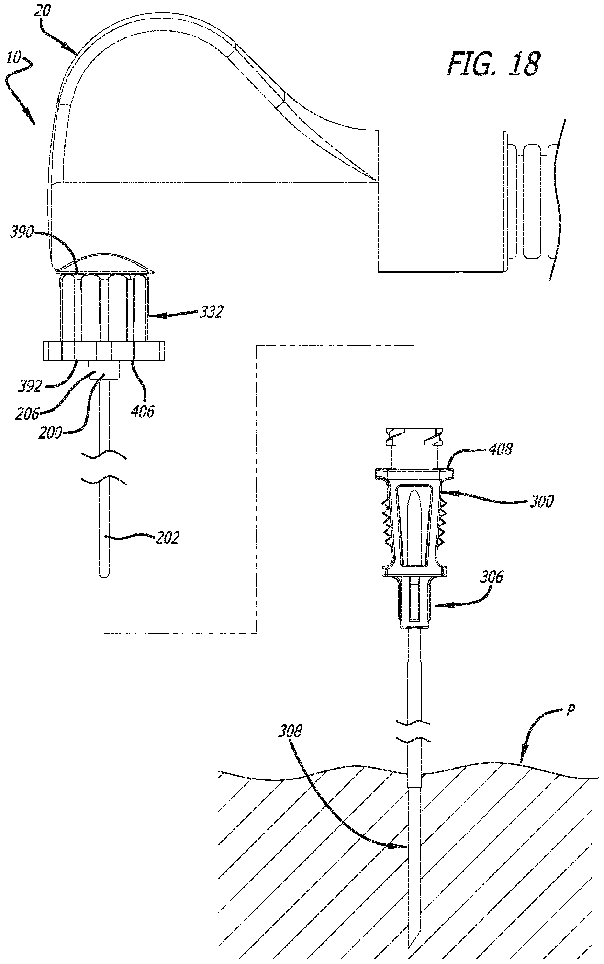

The introducer 300, as depicted in FIGS. 12 and 16, includes a proximal end 302 and a distal end 304 opposite from one another. As discussed below, the introducer 300 includes a handle portion 306 provided at the proximal end 302, and a cannula portion 308 extending from the handle portion 306 to the distal end 304. As depicted in FIGS. 17-20, the handle portion 306 is configured for attachment to the cryoprobe 10. Furthermore, as depicted in FIG. 18, a portion of the cannula portion 308 is configured for insertion into the body of the patient. As discussed below, the introducer 300 is configured to receive portions of the cryoprobe 10 such that during operation of the cryoprobe 10 all or a portion of the ice ball created by the cryoprobe is formed along a portion of the cannula portion 308. In addition to receiving portions of the cryoprobe 10, the introducer 300 can be used with a nerve stimulator (not shown) to verify the correct location of the introducer 300, and the introducer 300 can be used to facilitate introduction of anesthetic drugs to the area to be ablated. To illustrate, the nerve stimulator can be inserted into and through the introducer 300, and a syringe containing the anesthetic drugs can be attached to the introducer 300 and the anesthetic drugs can be introduced to the area to be ablated through the introducer 300.

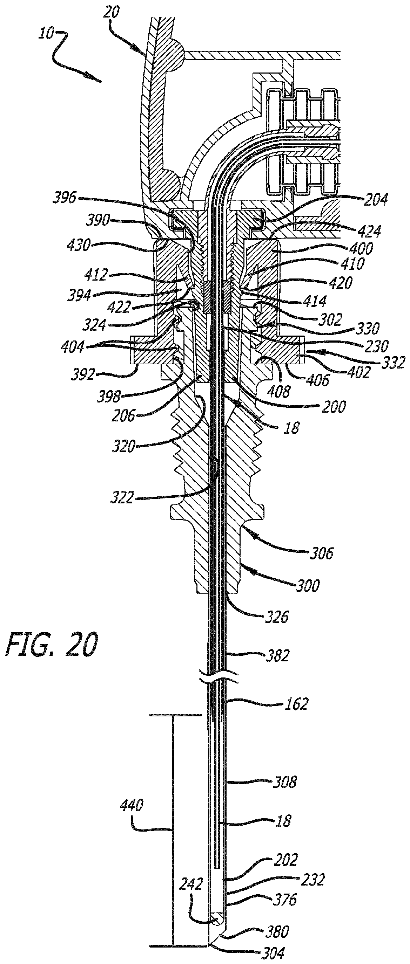

The handle portion 306 includes an end portion 310, a first flange portion, 312, a body portion 314, a second flange portion 316, and an extension portion 318. As depicted in FIGS. 16 and 20, the handle portion 306 also includes a first internal cavity 320 and a second internal cavity 322 extending therethrough. The first internal cavity 320 and the second internal cavity 322 communicate with one another, and the handle portion 306 includes a first opening 324 providing access to the first internal cavity 320, and a second opening 326 providing access to the second internal cavity 322. The first internal cavity 320 extends from the first opening 324 through the end portion 310, the first flange portion 312, and a portion of the body portion 314, and the second internal cavity 322 extends from the first internal cavity 320 to the second opening 326 through a portion of the body portion 314, the second flange portion 316, and the extension portion 318. As discussed below, the first internal cavity 320 is sized to receive a portion of the coupler portion 200, and the second internal cavity 322 facilitates attachment of the cannula portion 308 to the handle portion 306.

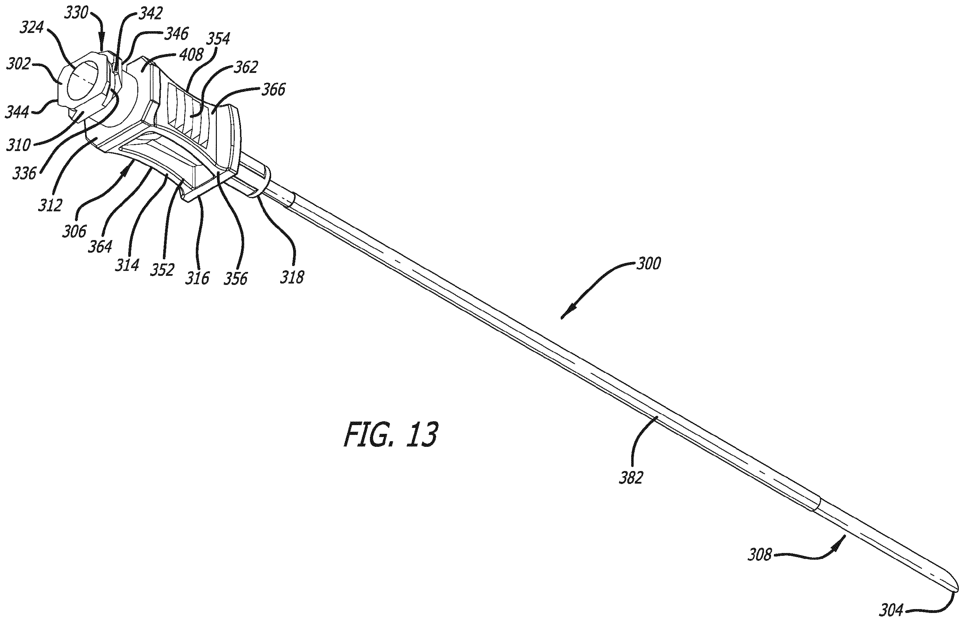

As depicted in FIGS. 12-15 and 17, the end portion 310 includes attachment structures 330 provided adjacent the proximal end 302. The attachment structures 330 are used to facilitate attachment of introducer 300 to a cap portion 332, and the cap portion 332 is used to facilitate attachment of the introducer 300 to the cryoprobe 10. The attachment structures 330 include channels 334 and 336 and detents 340 and 342. As depicted in FIGS. 12 and 13, the channel 334 and the detents 340 are formed on a first side 344 of the end portion 310, and the channel 336 and the detents 342 are formed on a second side 346 of the end portion 310. As discussed below, the channels 334 and 336 are angled to engage threads inside the cap portion 332, and portions on the inside of the cap portion 332 are captured in the channels 336 by the detents 340 and 342 to facilitate attachment of the introducer 300 and the cap portion 332 to one another.

As depicted in FIGS. 12-16, first flange portion 312 is positioned adjacent the end portion 310, and the body portion 314 is positioned adjacent the first flange portion 312. The first flange portion 312 and the body portion 314 can be configured to facilitate manipulation of the introducer 300 by a surgeon during surgery. To that end, indentations 350 and 352 can be formed on a first side 354 and a second side 356 of the body portion 314, respectively. Furthermore, gripping structures (such as, for example, fins 360 and 362, respectively) can be formed on a third side 364 and a fourth side 366 of the body portion 314. The indentations 350 and 352 and the gripping fins 360 serve in allowing a surgeon to grip the introducer 300, and in doing so, permit the surgeon to manipulate the introducer 300 into position with respect to the patient.

The second flange 316 can be used to prevent over-insertion of the cannula portion 308 into the human body. The second flange 316 includes a contact surface 370 that prevent the over-insertion of the cannula portion 308. Furthermore, the extension portion 318 is provided to support a portion of the cannula portion 308 therein. As discussed above, the second internal cavity 322 extends through a portion of the body portion 314 and the extension portion 318. The second internal cavity 322 can be sized to complement the external dimensions of an end portion 372. As such, the fit between the end portion 372 and the second internal cavity 322 can serve in attaching the cannula portion 308 to the handle portion 306.

The cannula portion 308, as depicted in FIGS. 12-20, extends outwardly from the handle portion 306. The cannula portion 308 is tubular, and includes a first end 374, a second end 376, and an internal cavity 378 extending between the first and second ends 374 and 376. The internal cavity 378 is sized to receive at least a portion of the probe shaft 202 therein. Furthermore, the end portion 372 extends from the first end 374 towards the second end 376, a tip 380 is provided at the second end 376, and insulation 382 is provided between the first and second ends 374 and 376. The tip 380 can be sharpened to facilitate penetration of the cannula portion 308 into the human body. As such, the tip 380 can cut through tissue to facilitate placement thereof adjacent the unwanted tissue that is to be ablated using the cryoprobe 10. When the nerve stimulator is used to verify the correct location of the introducer 300, the insulation 382 is used to insulate the patient from electrical nerve stimulation currents flowing through portions of the introducer 300. As such, the electrical nerve stimulation currents can be concentrated in the introducer 300 between the tip 380 and the insulation 382.

As discussed above, the introducer 300 is configured to receive portions of the probe shaft 202 and the coupler portion 200 therein. As depicted in FIGS. 16, 17, 18, and 20, the probe shaft 202 and the coupler portion 200 can be inserted through the introducer 300 so that a portion of the probe shaft 202 is ultimately received in the internal cavity 378 of the cannula portion 308, and so that a portion of the coupler portion 200 is received in the first internal cavity 320 of the handle portion 306. Furthermore, as discussed above, the attachment structures 330 and the cap portion 332 facilitate attachment of the introducer 300 to the cryoprobe 10. The configuration of the cap portion 332 has been adapted from a finger snap luer lock ring manufactured by Value Plastics, Inc.

The cap portion 332 includes a first end 390, and second end 392, and an internal cavity 394 extending between the first and second ends 390 and 392. Furthermore, the cap portion 332 includes a body portion 400 and a flange portion 402. As depicted in FIG. 20, the internal cavity 394 extends through the body portion 400 and the flange portion 402, the body portion 400 extends from the first end 390 to the flange portion 402, and the flange portion 402 extends from the body portion 400 to the second end 392. A first opening 396 in the body portion 400 provides access to the internal cavity 394 at the first end 390, and a second opening 398 in the flange portion 402 provides access to the internal cavity 394 at the second end 392. The internal cavity 394 includes threads 404 for engaging the channels 334 and 336 formed on the end portion 310. By engaging the channels 334 and 336 with the threads 404, the end portion 310 can be twisted into the internal cavity 394 through the second opening 398 in the cap portion 332. Detents (not shown) are provided in the internal cavity 394 near the end of the threads 404. Twisting of the end portion 310 and the cap portion 332 relative to one another ultimately pushes the detents provided in the interior cavity past the detents 340 and 342. The detents provided in the interior cavity are captured in the channels 334 and 336 by the detents 340 and 342. Such capture coincides with contact of an end surface 406 of the flange portion 402 with a surface 408 of the first flange portion 312. The introducer 300 and the cap portion 332 are attached to one another via these interactions.

As depicted in FIG. 20, the introducer 300 and the cap portion 332 can be attached to one another before or after the cap portion 332 is attached to the cryoprobe 10. As discussed above, the cap portion 332 is used to facilitate attachment of the introducer 300 to the cryoprobe 10. In addition to the threads 404, the internal cavity 394 of the cap portion 332 can include catches 410 and 412 used for attaching the cap portion 332 to the securing attachment of the coupler portion 200 of the probe portion 18. The catches 410 and 412 are attached to the cap portion 332 adjacent the first opening 396 at the first end 390. The catches 410 and 412 can be configured to deflect to allow a portion of the coupler portion 200 inserted through the opening 396 into the internal cavity 394 to pass thereby. To illustrate, insertion of the coupler portion 200 past the catches 410 and 412 causes the catches 410 and 412 to deflect away from one another. After a rim 414 formed on the coupler portion 200 is pushed past the catches 410 and 412, the catches 410 and 412 deflect toward one another, and in doing so, ends 420 and 422 of the catches 410 and 412, respectively, contact the rim 414. Such contact coincides with contact of an end surface 424 of the body portion 400 with a surface 430 of the head portion 20. The cryoprobe 10 and the cap portion 332 are attached to one another via these interactions. Thus, given that the introducer 300 and the cap portion 332 can be attached to one another, the introducer 300 can be attached to the cryoprobe 10 using the cap portion 332. The attachment facilitated by the cap portion 332 provides for a stable and firm connection between the cryoprobe 10 and the introducer 300 that prevents relative movement thereof during use.

As depicted in FIG. 18, a portion of the cannula portion 308 is configured for insertion into the body of the patient. The patient's body is identified by the letter P in FIG. 18. Rather than inserting the cryoprobe 10 directly into the patient P, the introducer 300 first can be positioned relative to the patient P, and the cryoprobe 10 second can be inserted into the introducer 300. In doing so, as depicted in FIGS. 16 and 20, the distal end 304 of the introducer 300 first can be positioned adjacent the unwanted tissue using the sharpened tip 380 to (if necessary) cut through tissue to facilitate such placement. And the second end 232 of the probe shaft 202 second can be positioned in the internal cavity 378 of the cannula portion 308 adjacent the distal end 304 of the introducer 300. As such, the tip 242 of the probe shaft 202, as depicted in FIG. 20, can be positioned adjacent the distal end 304 of the introducer 300.

After placement of the cryoprobe 10 relative to the introducer 300, the cryoprobe 10 can be operated to cool the end portion 250 (FIG. 10) of the probe shaft 202. The cryogenic temperatures generated at the end portion 250 can be used to cool the cannula portion 308, and an ice ball of frozen unwanted tissue can be formed around the cannula portion 308 at and adjacent the second end 376 thereof, rather than the end portion 250. As such, the cryogenic temperatures generated at the end portion 250 and the cannula portion 308 cooled thereby facilitates heat transfer from the adjacent tissue to freeze the unwanted tissue. Freezing in this manner serves to ablate the unwanted tissue.

The size and shape of the ice ball formed using the cryoprobe 10 and the introducer 300 is determined by the position of the insulating sheath 162. The ice ball forms around the cannula portion 308 at and adjacent the second end 376 from the tip 380 to adjacent the termination of the insulating sheath 162 in an area identified by the numeral 440 in FIG. 20. As such, if a larger or a smaller ice ball is needed, the cryoprobe 10 can be modified by adjusting the position of the termination of the insulating sheath 162. A larger ice ball would be formed by increasing the distance between the tip 380 and the termination of the insulating sheath 162, and a smaller ice ball would be formed by decreasing the distance between the tip 380 and the termination of the insulating sheath 162. Alternatively, an insulating sheath (not shown) can be provided on the interior or the exterior of the cannula portion 308 to facilitate sizing and shaping of ice ball formation. Like the insulating sheath 162, the distance between the tip 380 and the termination of an insulating sheath formed on the interior or the exterior of the cannula portion 308 can be adjusted to control the size and shape of the ice ball formed by the cryoprobe 10 and the introducer 300.

Because the probe shaft 202 does not have to bear the stress of directly being inserted into the patient, the probe shaft 202 can have less thickness and less strength than typical cryoprobes by using the introducer 300 with the cryoprobe 10.

Other embodiments of the invention will be apparent to those skilled in the art from consideration of the specification and practice of the invention disclosed herein. It is intended that the specification and examples be considered as exemplary only, with the scope of the invention being indicated by the following claims.

For Example, further aspects helping in the understanding of the invention may be as follows:

A combination of a cryoprobe, an introducer, and a cap portion used for attaching the cryoprobe and the introducer to one another, the combination comprising: the cryoprobe including a head portion, a probe shaft, and a cryogenic gas supply line, the probe shaft having a proximal end and a distal end, the probe shaft being attached to the head portion adjacent the proximal end, the cryogenic gas supply line extending through portions of the head portion and the probe shaft and terminating adjacent the distal end of the probe shaft, the cryogenic gas supply line during operation of the cryoprobe delivering cryogenic gas to the distal end of the probe shaft to facilitate cooling of a portion of the probe shaft; the introducer including a handle portion, a cannula portion, a first internal cavity, and a second internal cavity, the introducer having a proximal end and a distal end, the handle portion provided at the proximal end, and the cannula portion extending from the handle portion to the distal end, the handle portion including the first internal cavity extending therethrough, and the cannula portion including the second internal cavity extending therethrough, the first and second internal cavities communicating with one another and being sized to receive a portion of the probe shaft therethrough; and the cap portion facilitating the interconnection of the cryoprobe and the introducer, the cap portion including a proximal end, a distal end, a body portion provided adjacent the proximal end, a flange portion provided adjacent the distal end, and an internal cavity extending through the cap portion between the proximal end and the distal end thereof, the cap portion including a first attachment mechanism in the internal cavity adjacent the distal end used in attaching the handle portion to the cap portion, and the cap portion including a second attachment mechanism in the internal cavity adjacent the proximal end used in attaching the cryoprobe to the cap portion; wherein, when the probe shaft is inserted into the first and second internal cavities in the introducer, the distal end of the probe shaft is positionable adjacent the distal end of the introducer, and operation of the cryoprobe facilitates cooling of a portion of the cannula portion at the distal end of the introducer.

Further, a method of ablating unwanted tissue during surgery on a patient helping describing aspects of the invention is described in the following, the method including: providing an introducer partially insertable into the patient, the introducer including a proximal end, a distal end, a handle portion provided adjacent the proximal end, a cannula portion provided extending from the handle portion to the distal end, a first internal cavity extending through the handle portion, and a second internal cavity extending through the cannula portion, the first and second internal cavities communicating with one another; providing a cryoprobe including a head portion, a probe shaft, and a cryogenic gas supply line, the probe shaft having a proximal end and a distal end, the probe shaft being attached to the head portion adjacent the proximal end, the cryogenic gas supply line extending through portions of the head portion and the probe shaft and terminating adjacent the distal end of the probe shaft, inserting a portion of introducer into the patient; positioning the distal end of the introducer adjacent the unwanted tissue; inserting a portion the probe shaft through the first internal cavity and a portion of the second internal cavity of the introducer to position the distal end of the probe shaft in the cannula portion adjacent the distal end of the introducer; delivering cryogenic gas through the cryogenic gas supply line to the distal end of the probe shaft to facilitate cooling of the probe shaft; cooling the cannula portion at the distal end of the introducer; ablating the unwanted tissue by absorbing heat therefrom via the cooling of the cannula portion at the distal end of the introducer; removing the probe shaft from the portion of the second internal cavity and the first internal cavity of the introducer; and removing the introducer from the patient.

Further, the method may comprise cutting through tissue of the patient using a sharpened end of the introducer during positioning of the distal end thereof.

Further, the method may comprise freezing the unwanted tissue to form an ice ball around the cannula portion at the distal end of the introducer.

Further, the method may comprise providing insulation around a portion of the introducer.

Further, the method may comprise interconnecting the cryoprobe and the introducer using a cap portion, the cap portion including a first attachment mechanism for attaching the handle portion thereto, and the cap portion including a second attachment mechanism for attaching the cryoprobe thereto.

Further, the method may comprise providing a cap portion for interconnecting the cryoprobe and the introducer, attaching the handle portion and the cap portion to one another, and attaching the cryoprobe and the cap portion to one another.

Further, in the method, the cap portion may include an internal cavity extending between a proximal end and a distal end thereof, the cap portion including threads provided in the internal cavity thereof for engaging channels on the handle portion to attach the handle portion and the cap portion to one another, and the cap portion may include catches provided in the internal cavity thereof for engaging at least one rim on the cryoprobe to attach the cryoprobe and the cap portion.

## Claims

1. A combination of a cryoprobe (10) and an introducer (300) for use during surgery to ablate unwanted tissue of a patient, the combination comprising:
the cryoprobe (10) including a head portion (20), a probe shaft (202), and a cryogenic gas supply line, the probe shaft (202) having a proximal end (230) and a distal end (232), the probe shaft (202) being attached to the head portion (20) adjacent the proximal end (230), the cryogenic gas supply line extending through portions of the head portion (20) and the probe shaft (202) and terminating adjacent the distal end (232) of the probe shaft (202), the cryogenic gas supply line during operation of the cryoprobe (10) delivering cryogenic gas to the distal end (232) of the probe shaft (202) to facilitate cooling of a portion of the probe shaft (202); and
the introducer (300) including a handle portion (306), a cannula portion (308), a first internal cavity (320), and a second internal cavity (378), the introducer (300) having a proximal end (302) and a distal end (304), the handle portion (306) provided at the proximal end (302), and the cannula portion (308) extending from the handle portion (306) to the distal end (304) and including an open tip portion (380) sharpened to facilitate insertion of the introducer (300) into the patient, the handle portion (306) including the first internal cavity (320) extending therethrough, and the cannula portion (308) including the second internal cavity (378) extending therethrough, the first and second internal cavities (320, 378) communicating with one another and being sized to receive a portion of the probe shaft (202) therethrough;
wherein, when the probe shaft (202) is inserted into the first and second internal cavities (320, 378) in the introducer (300), the distal end (232) of the probe shaft (202) is positionable adjacent the distal end (304) of the introducer (300), and operation of the cryoprobe (10) facilitates cooling of a portion of the cannula portion (308) at the distal end (304) of the introducer (300); and
wherein the combination further includes a cap portion (332) facilitating the interconnection of the cryoprobe (10) and the introducer (300) to one another, the cap portion (332) including a first attachment mechanism (404) serving to attach the introducer (300) to the cap portion (332), a second attachment mechanism (410, 412) serving to attach the cryoprobe (10) to the cap portion (332), a body portion (400), a flange portion (402), and an internal cavity (394) extending through the body portion (400) and the flange portion (402), the internal cavity (394) being sized to receive portions of the cryoprobe (10) and the handle portion (306) therein, **characterized in that** the cap portion (332) is independently attachable to the introducer (300) and the cryoprobe (10).

2. The combination of claim 1, wherein a portion of the first internal cavity (320) extending therethrough is sized to receive a portion of the head portion (20) of the cryoprobe (10) therein.

3. The combination of claim 1, wherein the cap portion (332) includes a proximal end (390) and a distal end (392), the body portion (400) being provided at the proximal end (390), and the flange portion (402) being provided at the distal end (392), a first opening (396) into the internal cavity (394) of the cap portion (332) being provided through the body portion (400) at the proximal end (390) of the cap portion (332), and a second opening (398) into the internal cavity (394) of the cap portion (332) being provided through the flange portion (402) at the distal end (392) of the cap portion (332), the portion of the cryoprobe (10) being received through the first opening (396) into the internal cavity (394) of the cap portion (332), and the portion of the handle portion (306) being received through the second opening (398) into the internal cavity (394) of the cap portion (332).

4. The combination of claim 3, wherein the first attachment mechanism is formed as threads (404) in the internal cavity (394) of the cap portion (332) adjacent the distal end (392) thereof, and the handle portion (306) includes channels (334, 336) formed thereon for matingly engaging the threads (404) in the internal cavity (394), engagement of the threads (404) and the channels (334, 336) facilitating attachment of the introducer (300) to the cap portion (332).

5. The combination of claim 3 or 4, wherein the second attachment mechanism is formed as catches (410, 412) in the internal cavity (394) of the cap portion (332) adjacent the proximal end (390) thereof, and the cryoprobe (10) includes at least one rim (414) for engaging the catches (410, 412), engagement of the catches (410, 412) and the at least one rim (414) facilitating attachment of the cryoprobe (10) to the cap portion (332).

## Patentansprüche

1. Kombination aus einer Kryosonde (10) und einer Einführungsvorrichtung (300) zur Verwendung während einer Operation, um unerwünschtes Gewebe eines Patienten abzutragen, die Kombination umfassend:
die Kryosonde (10), die einen Kopfabschnitt (20), einen Sondenschaft (202) und eine kryogene Gasversorgungsleitung einschließt, wobei der Sondenschaft (202) ein proximales Ende (230) und ein distales Ende (232) aufweist, wobei der Sondenschaft (202) an dem Kopfabschnitt (20) benachbart dem proximalen Ende (230) angebracht ist, wobei sich die kryogene Gasversorgungsleitung durch Abschnitte des Kopfabschnitts (20) und des Sondenschafts (202) erstreckt und benachbart dem distalen Ende (232) des Sondenschafts (202) abschließt, wobei die kryogene Gasversorgungsleitung während des Betriebs der Kryosonde (10) kryogenes Gas an das distale Ende (232) des Sondenschafts (202) liefert, um ein Kühlen eines Abschnitts des Sondenschafts (202) zu erleichtern; und
die Einführungsvorrichtung (300), die einen Griffabschnitt (306), einen Kanülenabschnitt (308), einen ersten Innenhohlraum (320) und einen zweiten Innenhohlraum (378) einschließt, wobei die Einführungsvorrichtung (300) ein proximales Ende (302) und ein distales Ende (304) aufweist, wobei der Griffabschnitt (306) an dem proximalen Ende (302) bereitgestellt ist und sich der Kanülenabschnitt (308) von dem Griffabschnitt (306) zu dem distalen Ende (304) erstreckt und einen offenen Spitzenabschnitt (380) einschließt, der geschärft ist, um eine Einführung der Einführungsvorrichtung (300) in den Patienten zu erleichtern, wobei der Griffabschnitt (306) den ersten Innenhohlraum (320) einschließt, der sich dadurch hindurch erstreckt, und wobei der Kanülenabschnitt (308) den zweiten Innenhohlraum (378) einschließt, der sich dadurch hindurch erstreckt, wobei der erste und der zweite Innenhohlraum (320, 378) miteinander kommunizieren und bemessen sind, um einen Abschnitt des Sondenschafts (202) dadurch hindurch aufzunehmen;
wobei, wenn der Sondenschaft (202) in den ersten und den zweiten Innenhohlraum (320, 378) in die Einführungsvorrichtung (300) eingeführt wird, das distale Ende (232) des Sondenschafts (202) benachbart dem distalen Ende (304) der Einführungsvorrichtung (300) positionierbar ist und der Betrieb der Kryosonde (10) ein Kühlen eines Abschnitts des Kanülenabschnitts (308) am distalen Ende (304) der Einführungsvorrichtung (300) erleichtert; und
wobei die Kombination ferner einen Kappenabschnitt (332) einschließt, der die Verbindung der Kryosonde (10) und der Einführungsvorrichtung (300) miteinander erleichtert, wobei der Kappenabschnitt (332) einen ersten Befestigungsmechanismus (404) einschließt, der dazu dient, die Einführungsvorrichtung (300) an dem Kappenabschnitt (332) zu befestigen, einen zweiten Befestigungsmechanismus (410, 412), der dazu dient, die Kryosonde (10) an dem Kappenabschnitt (332) zu befestigen, einen Körperabschnitt (400), einen Flanschabschnitt (402) und einen Innenhohlraum (394), der sich durch den Körperabschnitt (400) und den Flanschabschnitt (402) erstreckt, wobei der Innenhohlraum (394) bemessen ist, um Abschnitte der Kryosonde (10) und des Griffabschnitts (306) darin aufzunehmen,
**dadurch gekennzeichnet, dass** der Kappenabschnitt (332) unabhängig an der Einführungsvorrichtung (300) und der Kryosonde (10) anbringbar ist.

2. Kombination nach Anspruch 1, wobei ein Abschnitt des ersten Innenhohlraums (320), der sich dadurch erstreckt, bemessen ist, um einen Abschnitt des Kopfabschnitts (20) der Kryosonde (10) darin aufzunehmen.

3. Kombination nach Anspruch 1, wobei der Kappenabschnitt (332) ein proximales Ende (390) und ein distales Ende (392) einschließt, wobei der Körperabschnitt (400) an dem proximalen Ende (390) bereitgestellt ist, und wobei der Flanschabschnitt (402) an dem distalen Ende (392) bereitgestellt ist, wobei eine erste Öffnung (396) in den Innenhohlraum (394) des Kappenabschnitts (332) durch den Körperabschnitt (400) am proximalen Ende (390) des Kappenabschnitts (332) bereitgestellt ist, und eine zweite Öffnung (398) in den Innenhohlraum (394) des Kappenabschnitts (332) durch den Flanschabschnitt (402) am distalen Ende (392) des Kappenabschnitts (332) bereitgestellt ist, wobei der Abschnitt der Kryosonde (10) durch die erste Öffnung (396) in den Innenhohlraum (394) des Kappenabschnitts (332) aufgenommen ist und der Abschnitt des Griffabschnitts (306) durch die zweite Öffnung (398) in den Innenhohlraum (394) des Kappenabschnitts (332) aufgenommen ist.

4. Kombination nach Anspruch 3, wobei der erste Befestigungsmechanismus als Gewinde (404) im Innenhohlraum (394) des Kappenabschnitts (332) benachbart dem distalen Ende (392) davon ausgebildet ist, und der Griffabschnitt (306) Kanäle (334, 336) einschließt, die zum passenden Eingreifen der Gewinde (404) in den Innenhohlraum (394) darauf ausgebildet sind, wobei der Eingriff der Gewinde (404) und der Kanäle (334, 336) eine Befestigung der Einführungsvorrichtung (300) an dem Kappenabschnitt (332) erleichtern.

5. Kombination nach Anspruch 3 oder 4, wobei der zweite Befestigungsmechanismus als Schnappverschlüsse (410, 412) in dem Innenhohlraum (394) des Kappenabschnitts (332) benachbart dem proximalen Ende (390) davon ausgebildet ist und die Kryosonde (10) mindestens einen Rand (414) zum Eingreifen in die Schnappverschlüsse (410, 412) einschließt, wobei der Eingriff der Schnappverschlüsse (410, 412) und des mindestens einen Randes (414) eine Befestigung der Kryosonde (10) an dem Kappenabschnitt (332) erleichtern.

## Revendications

1. Combinaison d'une cryosonde (10) et d'un introducteur (300) pour une utilisation pendant une intervention chirurgicale pour ablater un tissu indésirable d'un patient, la combinaison comprenant :
la cryosonde (10) comportant une partie tête (20), un arbre de sonde (202), et une conduite d'alimentation en gaz cryogénique, l'arbre de sonde (202) ayant une extrémité proximale (230) et une extrémité distale (232), l'arbre de sonde (202) étant fixé à la partie tête (20) à proximité de l'extrémité proximale (230), la conduite d'alimentation en gaz cryogénique s'étendant à travers des parties de la partie tête (20) et l'arbre de sonde (202) et se terminant à proximité de l'extrémité distale (232) de l'arbre de sonde (202), la conduite d'alimentation en gaz cryogénique pendant le fonctionnement de la cryosonde (10) délivrant du gaz cryogénique à l'extrémité distale (232) de l'arbre de sonde (202) pour faciliter le refroidissement d'une partie de l'arbre de sonde (202) ; et
l'introducteur (300) comportant une partie poignée (306), une partie canule (308), une première cavité interne (320) et une seconde cavité deinterne (378), l'introducteur (300) ayant une extrémité proximale (302) et une extrémité distale (304), la partie poignée (306) étant disposée au niveau de l'extrémité proximale (302), et la partie canule (308) s'étendant depuis la partie poignée (306) jusqu'à l'extrémité distale (304) et comportant une partie pointe ouverte (380) aiguisée pour faciliter l'insertion de l'introducteur (300) dans le patient, la partie poignée (306) comportant la première cavité interne (320) s'étendant à travers celle-ci, et la partie canule (308) comportant la seconde cavité interne (378) s'étendant à travers celle-ci, les première et seconde cavités internes (320, 378) communiquant l'une avec l'autre et étant dimensionnées pour recevoir une partie de l'arbre de sonde (202) à travers celles-ci ;
dans lequel, lorsque l'arbre de sonde (202) est inséré dans les première et seconde cavités internes (320, 378) dans l'introducteur (300), l'extrémité distale (232) de l'arbre de sonde (202) peut être positionnée à proximité de l'extrémité distale (304) de l'introducteur (300), et le fonctionnement de la cryosonde (10) facilite le refroidissement d'une partie de la partie canule (308) au niveau de l'extrémité distale (304) de l'introducteur (300) ; et
dans lequel la combinaison comporte en outre une partie capuchon (332) facilitant l'interconnexion de la cryosonde (10) et de l'introducteur (300) l'un à l'autre, la partie capuchon (332) comportant un premier mécanisme de fixation (404) servant à fixer l'introducteur (300) à la partie capuchon (332), un second mécanisme de fixation (410, 412) servant à fixer la cryosonde (10) à la partie capuchon (332), une partie corps (400), une partie bride (402) et une cavité interne (394) s'étendant à travers la partie corps (400) et la partie bride (402), la cavité interne (394) étant dimensionnée pour recevoir des parties de la cryosonde (10) et de la partie poignée (306) à l'intérieur de celle-ci,
**caractérisé en ce que** la partie capuchon (332) peut être fixée indépendamment à l'introducteur (300) et à la cryosonde (10).

2. Combinaison selon la revendication 1, dans laquelle une partie de la première cavité interne (320) s'étendant à travers celle-ci est dimensionnée pour recevoir une partie de la partie tête (20) de la cryosonde (10) à l'intérieur de celle-ci.

3. Combinaison selon la revendication 1, dans laquelle la partie capuchon (332) comporte une extrémité proximale (390) et une extrémité distale (392), la partie corps (400) étant disposée au niveau de l'extrémité proximale (390), et la partie bride (402) étant disposée au niveau de l'extrémité distale (392), une première ouverture (396) dans la cavité interne (394) de la partie capuchon (332) étant disposée à travers la partie corps (400) au niveau de l'extrémité proximale (390) de la partie capuchon (332), et une seconde ouverture (398) dans la cavité interne (394) de la partie capuchon (332) étant disposée à travers la partie bride (402) au niveau de l'extrémité distale (392) de la partie capuchon (332), la partie de la cryosonde (10) étant reçue à travers la première ouverture (396) dans la cavité interne (394) de la partie capuchon (332), et la partie de la partie poignée (306) étant reçue à travers la seconde ouverture (398) dans la cavité interne (394) de la partie capuchon (332).

4. Combinaison selon la revendication 3, dans laquelle le premier mécanisme de fixation est formé en tant que filetages (404) dans la cavité interne (394) de la partie capuchon (332) à proximité de l'extrémité distale (392) de celle-ci, et la partie poignée (306) comporte des canaux (334, 336) formés sur celle-ci pour venir en prise par accouplement avec des filetages (404) dans la cavité interne (394), une mise en prise des filetages (404) et des canaux (334, 336) facilitant la fixation de l'introducteur (300) à la partie capuchon (332).

5. Combinaison selon la revendication 3 ou 4, dans laquelle le second mécanisme de fixation est formé en tant que crochets (410, 412) dans la cavité interne (394) de la partie capuchon (332) à proximité de l'extrémité proximale (390) de celui-ci, et la cryosonde (10) comportant au moins un rebord (414) pour venir en prise avec les crochets (410, 412), la mise en prise des crochets (410, 412) et de l'au moins un rebord (414) facilitant la fixation de la cryosonde (10) à la partie capuchon (332).
